# EUROPEAN PATENT SPECIFICATION

(11) **EP 4 011 859 B1**
(45) Date of publication and mention of the grant of the patent: **30.08.2023**
(21) Application number: 20212336.0
(22) Date of filing: 08.12.2020
(51) Int. Cl.: C07F 15/00, C07C 51/00

(54) **METHOD FOR CHEMICAL STORAGE OF HYDROGEN**
VERFAHREN ZUR CHEMISCHEN SPEICHERUNG VON WASSERSTOFF
PROCÉDÉ DE STOCKAGE CHIMIQUE D'HYDROGÈNE

(43) Date of publication of application: 15.06.2022
(73) Proprietor: APEX ENERGY TETEROW GMBH, 17166 Teterow (DE)
(72) Inventor: Sponholz, Peter, 18055 Rostock (DE); Sang, Rui, Liao cheng Province: Shandong (CN); Jackstell, Ralf, 18106 Rostock (DE); Junge, Henrik, 18147 Rostock (DE); Beller, Matthias, 18211 Nienhagen (DE)
(74) Representative: Gulde & Partner

(56) References cited:
- WO-A1-2015/083007
- US-A1- 2014 255 296
- US-A1- 2017 014 817
- ZHIJUN WANG ET AL: "Unprecedentedly High Formic Acid Dehydrogenation Activity on an Iridium Complex with an N , N '-Diimine Ligand in Water", CHEMISTRY - A EUROPEAN JOURNAL, vol. 21, no. 36, 1 September 2015 (2015-09-01), pages 12592-12595, XP055405460, ISSN: 0947-6539, DOI: 10.1002/chem.201502086

## Description

### Field of the Invention

The invention relates to the field of chemical storage of hydrogen, in particular the development of efficient and selective catalyst systems.

### Technological Background

In the last decades, the importance of renewable energies from wind and sunlight has steadily increased. However, to ensure a sufficient and sustainable energy supply, efficient solutions for storing the generated electrical energy are required to compensate for the fluctuating availability of wind and sunlight. In that context, excess energy from windmills and photovoltaic plants is increasingly being stored in the form of hydrogen gas using conventional water electrolysis.

Although hydrogen gas could offer the opportunity of an on-demand energy supply, the safe storage of this highly flammable gas remains a challenging task. Efficient handling and storage methods are therefore key factors that need to be addressed for the successful introduction of H₂ into our energy infrastructure.

The first example of a reversible CO₂-based H₂ storage cycle dates to 1994. Leitner *et al.* demonstrated that the formic acid adduct produced in the presence of [Rh(COD)Cl]₂/DPPB in an acetone/NEt₃ mixture under 40 bar H₂/CO₂ at ambient temperature, could be directly dehydrogenated upon release of the overpressure (Leitner et al., J. Organomet. Chem. 1994, 475, 257-266). Till that time, the hydrogenation of C-based compounds either to formic acid or methanol has been extensively investigated and outstanding activities were achieved.

The use of homogeneous catalysts based on noble metals, such as iridium- and ruthenium-PNP pincer type complexes, already enables the hydrogenation of carbon dioxide to formic acid or formate with activities (TOF) of up to 1,100,000 and 73,000 as well as productivities (TON) of up to 3,500,000 and 250,000, respectively (Tanaka et al., Organometallics 2011, 30, 6742-6750 for Ir, Filonenko et al., ChemCatChem 2014, 6, 1526-1530 for Ru). For the reverse dehydrogenation of formic acid, iridium complexes with *N*,*N*-bidentate ligands possess outstanding activity and durability with a TOF as high as 487,000 h⁻¹ and an TON up to 2,400,000 (Wang et al., Chem. Eur. J. 2015, 21, 12592-12595).

Further, methanol is considered as a promising chemical energy carrier due to its relatively high hydrogen content, low volatility at ambient conditions and the possibility of using the existing infrastructure for its storage and transportation (Olah et al., Angew. Chem. Int. Ed. 2013, 52, 104-107). While homogeneous catalysts in COz-hydrogenation to methanol (> 9,500 turnovers) are not yet as productive (Kar et al., J. Am. Chem. Soc. 2019, 141, 3160-3170), the use of heterogeneous catalysts, e.g. in the "George Olah CO₂ to Renewable Methanol Plant" in Iceland (Olah, Angew. Chem. Int. Ed. 2013, 52, 104-107) may yield activities up to 3,500 tons per year. Here, the reverse hydrogen release from methanol is mainly realized by steam reforming processes, which usually require higher temperatures (200-300 °C) with heterogeneous catalysts (Palo et al., Chem. Rev. 2007, 107, 3992-4021; Sá et al., Appl. Catal. B 2010, 99, 43-57). The drawback of such high temperatures in the steam reforming of methanol lays in a considerable carbon monoxide formation, which poisons the electrodes when applied in Reformed Methanol Fuel Cells (RMFC). One approach to solve this problem is the so-called aqueous phase reforming (APR) of methanol. Examples may be found in WO 2015/083007 A1, US 2014/0255296 A1, US 2017/0014817 A1 or XP 55405460 A.

However, there is a continuing need to develop more effective and selective catalyst systems for the reversible chemical storage of hydrogen, especially in view of reduced carbon monoxide formation. In general, the objectives of a storage system are a high safety standard as well as satisfying volumetric and gravimetric H₂ capacities, reasonable refuelling times and an adequate lifespan. Further, it must be energetically efficient and available at reasonable costs.

### Summary of Invention

The objective of the invention is solved, and the disadvantages of the prior art are overcome, at least partially, by the method for a reversible chemical storage of hydrogen according to the appended claims.

An aspect of the disclosure relates to a method for a reversible chemical storage of hydrogen. Said method comprises a hydrogenation step in which hydrogen is converted into at least formic acid as to chemically store the hydrogen, and a (subsequent) dehydrogenation step, in which hydrogen is released from a mixture comprising or consisting of C₁-C₁₈ alcohol, formic acid and optionally water. In a preferred embodiment, the hydrogenation step comprises contacting hydrogen with carbon dioxide in the presence of a hydrogenation catalyst in order to effect the storage of hydrogen by forming a mixture of formic acid and methanol.

The dehydrogenation step comprises converting a mixture comprising or consisting of C₁-C₁₈ alcohol, formic acid and optionally (stoichiometric amounts of) water into hydrogen and carbon dioxide in the presence of a base and a transition-metal hydride catalyst, wherein the transition-metal hydride catalyst may be formed *in situ* from a precursor of the transition-metal hydride catalyst, or a heterogeneous transition-metal catalyst. The transition-metal hydride catalyst comprises a metal-ion selected from the group consisting of Ru, Ir, Mn, Co, Mo, Fe and Rh and at least one hydride ligand coordinated to the metal-ion. Further the transition-metal hydride catalyst comprises a tridentate ligand, or one or two of a bidentate ligand, or a tetradentate *PPPP*-chelating ligand, which is coordinated to the metal-ion to form the transition-metal hydride catalyst. The tridentate ligand is selected from the group consisting of a *PNP*-chelating ligand, a *PNN*-chelating ligand, a *PCP*-chelating ligand, an *NCN*-chelating ligand, a *PCN*-chelating ligand, a *PPP*-chelating ligand and an *NNN*-chelating ligand. The tetradentate ligand is a *PPPP*-chelating ligand. Further, the bidentate ligand is selected from a *PP*-chelating ligand or an *NN*-chelating ligand.

Not falling under the protection scope of the present invention, a second aspect of the disclosure refers to a reversible chemical storage system for hydrogen construed for performing the method described above.

Further preferred embodiments of the invention result from the following description.

### Brief Description of the Drawings

- Figure 1: illustrates the carbon dioxide-based hydrogen storage in a formic acid/alcohol mixture cycle.

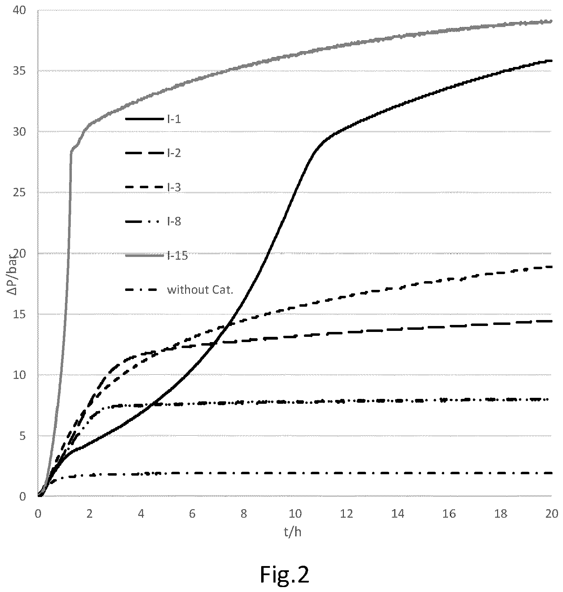

- Figure 2: shows the activity of transition-metal hydride catalysts 1-1 to I-3, I-8 and I-15 in the acceptorless dehydrogenation of a mixture of methanol and formic acid. The pressure increase (p/[bar]) corresponds to the catalytically induced release of hydrogen and carbon dioxide over time (t/[h]).
- Figure 3: shows the activity of transition-metal hydride catalysts I-1 in the acceptorless dehydrogenation of a mixture of methanol and formic acid compared to formic acid and methanol alone. The pressure increase (p/[bar]) corresponds to the catalytically induced release of hydrogen and carbon dioxide over time (t/[h]).
- Figure 4: shows the activity of transition-metal hydride catalysts I-1 in the acceptorless dehydrogenation of a mixture of methanol and formic acid compared to methyl formate. The pressure increase (p/[bar]) corresponds to the catalytically induced release of hydrogen and carbon dioxide over time (t/[h]).
- Figure 5: shows the influence of various alcohols on the acceptorless dehydrogenation in a mixture with formic acid and in the presence of catalyst I-1. The pressure increase (p/[bar]) corresponds to the catalytically induced release of hydrogen and carbon dioxide over time (t/[h]).
- Figure 6: shows the influence of various ratios of formic acid (FA) and methanol on the acceptorless dehydrogenation in the presence of catalyst I-1. The pressure increase (p/[bar]) corresponds to the catalytically induced release of hydrogen and carbon dioxide over time (t/[h]).
- Figure 7: shows the influence of starting pH on the acceptorless dehydrogenation in a mixture with formic acid and in the presence of catalyst I-1. The pressure increase (p/[bar]) corresponds to the catalytically induced release of hydrogen and carbon dioxide over time (t/[h]).
- Figure 8: shows the influence of the water content on the acceptorless dehydrogenation in a mixture with formic acid and in the presence of catalyst I-1. The pressure increase (p/[bar]) corresponds to the catalytically induced release of hydrogen and carbon dioxide over time (t/[h]).

### Detailed Description of the Invention

Hereinafter, a method for the reversible chemical storage of hydrogen will be described in detail, and with reference to specific embodiments.

The method comprises a hydrogenation step and a dehydrogenation step in order to chemically store and release hydrogen on demand.

The hydrogenation step comprises contacting hydrogen with carbon dioxide in the presence of a hydrogenation catalyst to effect storage of hydrogen by forming at least formic acid. The hydrogenation step may yield a mixture of formic acid and methanol by e.g. further hydrogenation of formic acid to methanol in the presence of a hydrogenation catalyst. The aforementioned hydrogenation step may yield the formic acid by contacting hydrogen with carbon dioxide in the presence of a (first) hydrogenation catalyst and methanol by contacting hydrogen with formic acid in the presence of a (second) hydrogenation catalyst that differs from the (first) hydrogenation catalyst used for the formation of formic acid. In another embodiment, the hydrogenation step may further comprise contacting hydrogen with carbon dioxide in the presence of a hydrogenation catalyst, which catalyses the direct formation of methanol (without intermediate formic acid formation).

The dehydrogenation step comprises converting a mixture comprising or consisting of a C₁-C₁₈ alcohol, formic acid and optionally (stoichiometric amounts of) water into hydrogen and carbon dioxide in the presence of a base and either a transition-metal hydride catalyst, wherein the transition-metal hydride catalyst may be formed in situ from a precursor thereof, or a heterogeneous transition-metal catalyst. For said mixture in the dehydrogenation step, at least one of the C₁-C₁₈ alcohol and formic acid is derived from the hydrogenation step. The formic acid may be formed *in situ* from a formate salt. Non-limiting examples for the formate salt include sodium formate, potassium formate and ammonium formate.

In one embodiment, in the dehydrogenation step the C₁-C₂ alcohol is derived from the hydrogenation step and the formic acid is formed *in situ* from a formate salt, which is added to the mixture together with equimolar or catalytic amounts of a stronger acid. Examples for such stronger acid may be hydrochloric acid or sulfuric acid.

In another embodiment, in the dehydrogenation step the formic acid is derived from the hydrogenation step and the C₁-C₁₈ alcohol is added to the mixture. The C₁ - C₁₈ alcohol may be a C₁ - C₆ alcohol. Preferably, the C₁ - C₁₈ alcohol is methanol or ethanol.

The pH of the mixture may be in range of 3.9 to 14, preferably in the range 7.3 to 14 in order to provide optimum activity of the transition-metal hydride catalyst.

The (homogeneous) transition-metal hydride catalyst comprises a metal-ion selected from the group consisting of Ru, Ir, Mn, Co, Mo, Fe and Rh and at least one hydride ligand coordinated to the metal-ion. The transition-metal hydride catalyst further comprises one or two of a polydentate ligand coordinated to the metal-ion, which is i) a tridentate ligand selected from the group consisting of a *PNP*-chelating ligand, a *PNN*-chelating ligand, a *PCP*-chelating ligand, an *NCN-*chelating ligand, a *PCN*-chelating ligand, a *PPP*-chelating ligand and an *NNN-*chelating ligand, or ii) a bidentate ligand selected from a *PP*-chelating ligand or an *NN-*chelating ligand, or iii) a tetradentate *PPPP*-chelating ligand. In the case of the tridentate ligands and the tetradentate ligands it is preferably one ligand that coordinates (complexes) the metal ion. In case of a bidentate ligand it is one or two bidentate ligands coordinated to the metal ion. The transition-metal hydride catalyst may further comprise a halide coordinated to the metal-ion.

In one embodiment, the transition-metal hydride catalyst comprises a metal-ion selected from the group consisting of Ru, Ir, Mn, and Fe. Preferably, the metal-ion is selected from the group consisting of Ru, Ir and Mn. In another preferred embodiment the metal-ion is Ru or Ir.

The dehydrogenation step in presence of a precursor of the transition-metal hydride catalyst is understood in terms of forming the transition-metal hydride catalyst in situ by means of the precursor. The precursor may comprise a metal ion source selected from at least one of the group consisting of Ru, Ir, Mn, Co, Mo, Fe and Rh ion sources, and 1 to 10 molar equivalents of the bidentate ligand or the tridentate ligand or the tetradentate ligand as introduced above. Preferably, the precursor comprises the metal ion source and 2 to 6 molar equivalents of the bidentate ligand or the tridentate ligand or the tetradentate ligand.

The metal ion source may be a transition metal complex capable of forming the transition-metal hydride catalyst via ligand exchange reaction with a hydride ligand and/or with the abovementioned polydentate ligands. Non limiting examples for said metal ion source may include dichloro(benzene)ruthenium(ll) dimer (CAS: 37366-09-9), (acetylacetonato)-dicarbonyl-rhodium(I) (CAS: 14874-82-9) and iron(II) tetrafluoroborate hexahydrate (CAS: 13877-16-2). In a preferred embodiment the metal ion source includes Ru, Ir, Mn, and Fe ion sources. Preferably, the metal ion source is dichloro(benzene)ruthenium(II) dimer or iron(II) tetrafluoroborate hexahydrate. The transition-metal hydride catalyst may be formed in situ from the precursor via oxidative addition of hydrogen or dehydrohalogenation.

In this disclosure the hydrogenation catalyst may be any literature-known heterogeneous or homogeneous catalyst capable of catalysing the hydrogenation reaction with carbon dioxide to provide formic acid . Preferably, the hydrogenation catalyst may be capable of catalysing the formation of formic acid from the hydrogenation reaction and may further catalyse a reaction from formic acid to methanol. An example for a suitable hydrogenation catalyst may be found in WO 2017093782 A1.

Embodiments of the transition-metal hydride catalyst used in the dehydrogenation step will be described in the following:
As mentioned above the transition-metal hydride catalyst comprises a tridentate ligand, or one or two of a bidentate ligand, or a tetradentate PPPP-chelating ligand, which is coordinated to the metal-ion centre of the catalyst.

In one embodiment, the tridentate ligand of the transition-metal hydride catalyst may be a *PNP* chelating ligand or a *PNN* chelating ligand.

In a preferred embodiment, the tridentate ligand of the transition-metal hydride catalyst is a *PNP* chelating ligand according to formula (II): 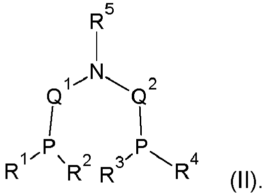 Q¹ and Q² in formula (II) are independently selected from the group consisting of C₂-C₆ alkylene group and C₅-C₇ cycloalkylene group. In formula (II), the substituents at the phosphor moieties R¹, R², R³ and R⁴ are independently selected from at least one of the group consisting of C₁-C₆ alkyl group, C₃-C₇ cycloalkyl group, C₆-C₁₄ aryl group, C₂-C₉ heteroaryl group and benzyl. Preferably, R¹, R², R³ and R⁴ are independently selected from the group consisting of *iso-*propyl, *tert*-butyl*,* phenyl and ethyl. R⁵ is selected from the group consisting of H, C₁ - C₈ alkyl group and C₃-C₇ cycloalkyl group.

In another preferred embodiment, Q¹ and Q² in formula (II) are independently selected from at least one of the group consisting of C₂-C₆ alkylene group and C₅-C₇ cycloalkylene group; the substituents at the phosphor moieties R¹, R², R³ and R⁴ are independently selected from at least one of the group consisting of C₁-C₆ alkyl group, C₃-C₇ cycloalkyl group, C₆-C₁₄ aryl group, C₂-C₉ heteroaryl group and benzyl; R¹, R², R³ and R⁴ are independently selected from the group consisting of *iso*-propyl, *tert*-butyl, phenyl and ethyl; and R⁵ is selected from the group consisting of H, methyl, ethyl, *iso-*propyl, *tert*-butyl, cylcohexyl.

In yet another preferred embodiment, Q¹ and Q² in formula (II) are independently selected from the group consisting of C₂-C₆ alkylene group and C₅-C₇ cycloalkylene group; the substituents at the phosphor moieties R¹, R², R³ and R⁴ are independently selected from the group consisting of C₁-C₆ alkyl group, C₃-C₇ cycloalkyl group, C₆-C₁₄ aryl group, C₂-C₉ heteroaryl group and benzyl; R¹, R², R³ and R⁴ are independently selected from the group consisting of iso-propyl, tert-butyl, phenyl and ethyl; and R⁵ is H or methyl.

Preferred embodiments of the transition-metal hydride catalyst are selected from at least one of the group of complexes according to formulae I-1 to I-9:

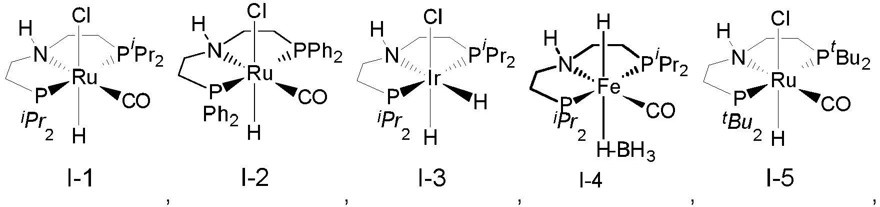

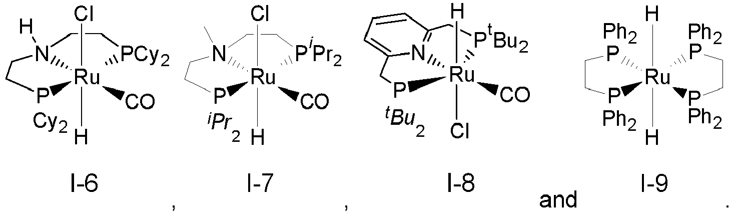

In another embodiment, the transition-metal hydride catalyst may be formed in situ by a precursor selected from the at least one of group of compounds according to formulae I-10 to I-15:

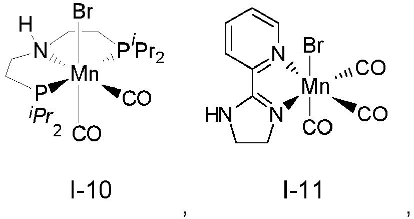

Fe(BF)•6H₂O/PP3 I-12 ,

[RuCl₂(benzene)]₂/Triphos¹ I-13 ,

[RuCl₂(benzene)]₂/Triphos² I-14

and

[RuCl₂(benzene)]₂/dppe I-15 ,

wherein

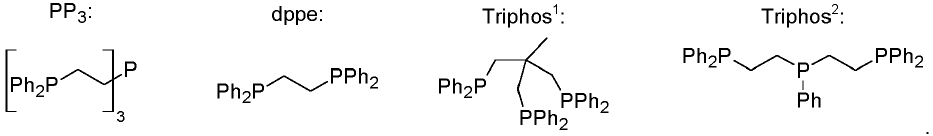

Herein the precursor may comprise a metal ion source [M] and a polydentate ligand [L], shown above as [M]/[L], e.g. RuCl₂(benzene)/dppe. The precursor may comprise 1 to 6 molar equivalents of the polydentate ligand [L] referred to the metal ion source [M]. In a preferred embodiment, the precursor may comprise 6 molar equivalents of the polydentate ligand [L] referred to the metal ion source [M].

In a preferred embodiment, the transition-metal hydride catalysts may be selected from the group complexes consisting of I-1, I-2 and I-3 due to high catalytic activity and low carbon monoxide formation.

The transition-metal hydride catalyst may be present at 0.001 mol% to 0.01 mol%, preferably 0.002 mol% to 0.006 mol% (referred to 1 mol of the formic acid) providing high catalytic efficiency and thus economic benefits.

In another embodiment, the dehydrogenation step is performed in presence of a heterogeneous transition-metal catalyst which comprises at least one of a metal selected from the group consisting of ruthenium, palladium, platinum and cobalt.

Preferably, the dehydrogenation step is performed in presence of a heterogeneous transition-metal catalyst selected from the group consisting of ruthenium on Al₂O₃ (5 wt.% Ru), palladium on Al₂O₃ (5 wt.% Pd), platinum on Al₂O₃ (5 wt.% Pt) and CoNₓ on carbon (2.2 wt.% CoNₓ). Preferably, the heterogeneous transition-metal catalyst is ruthenium on Al₂O₃ (5 wt.% Ru) as it provides best catalytic hydrogen release combined with low carbon monoxide formation.

The presence of a base in the dehydrogenation step may activate the transition-metal hydride catalyst by deprotonation at the pincer ligand (e.g. deprotonation of NH-group) and formation of alkali halide salts with the halide ligand of the transition-metal hydride catalyst, i.e. creating a vacant coordination site for the substrate at the transition metal.

In one embodiment the base is selected from the group consisting of NaH, LiOH, NaOH, KOH, CsOH, LiOEt, NaOEt, KOEt, KOC(CH₃)₃, LiHCO₃, NaHCO₃, KHCO₃, CsHCO₃, Li₂CO₃, Na₂CO₃, K₃PO₄, K₂CO₃ and C_{S2}CO₃. Preferably, the base is selected from at least one of the group consisting of KOH, LiOH, CsOH, NaOH and K₃PO₄, as these bases provide an optimal catalyst activation and low carbon monoxide formation. In another preferred embodiment the base is KOH. The K₃PO₄ used herein may be anhydrous K₃PO₄ (CAS: 7778-53-2). The base may be present at 5 mol% to 35 mol% referred to the formic acid. Preferably, the base is present at 11 mol% to 24 mol% referred to the formic acid in order to maximize the catalytic hydrogen release and to minimize carbon monoxide formation.

In a preferred embodiment, the molar ratio of the mixture according to formic acid : C₁-C₁₈ alcohol : water may be 0.9 to 1.1 : 0.5 to 2 : 0 to 2, preferably 0.9 to 1.1 : 0.9 to 2 : 0 to 2. More preferably, the molar ratio of the mixture according to C₁-C₁₈ alcohol: formic acid : water is 0.9 to 1.1 : 0.9 to 1.1 : 0.9 to 1.1.

In another embodiment, water is present at 0 to 2.0 equivalents per mol formic acid. Preferably, water is present at 0.5 to 1.5 equivalents per mol formic acid in order to maximize the catalytic hydrogen release and to minimize carbon monoxide formation. Most preferred, water is present at 0.9 to 1.1 equivalents per mol formic acid.

In another embodiment, the dehydrogenation step is performed at a reaction temperature of 20°C to 150°C, which represents a considerable energy saving compared to the state of art, which requires reaction temperatures above 200°C. Preferably, dehydrogenation step is performed at a reaction temperature of 40°C to 90°C in order to maximize the catalytic hydrogen release and to minimize carbon monoxide formation.

In one embodiment, the hydrogenation catalyst corresponds to the transition-metal hydride catalyst or to the heterogeneous transition-metal catalyst, that is, the hydrogenation step and dehydrogenation step may be performed in presence of the same catalyst.

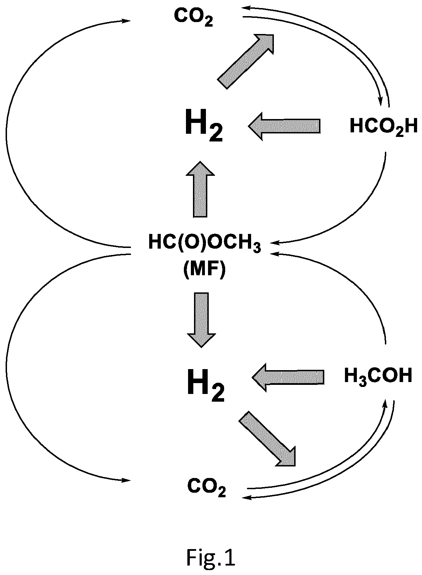

Figure 1, shows an embodiment of the present invention in form of a catalytic cycle including the chemical storage of hydrogen (hydrogenation) and release of hydrogen (dehydrogenation). Both reaction steps, hydrogenation and dehydrogenation, are catalytically driven in the presence of a suitable catalyst as will be described in the following. In figure 1, the upper sub-cycle includes the hydrogenation of carbon dioxide in the presence of a hydrogenation catalyst to yield formic acid, whereas the lower sub-cycle includes the hydrogenation of carbon dioxide in the presence of a hydrogenation catalyst to yield methanol. Herein the hydrogenation catalyst used for the hydrogenation reactions in both sub-cycles is preferably the same catalyst, but may also be of different kind. The catalytically formed formic acid and methanol may partially react to form methyl formate *in situ.*

In figure 1, the release of hydrogen (and carbon dioxide) is realized by catalytically driven dehydrogenation reaction of methanol, formic acid and *in situ* formed methyl formate in the presence of a dehydrogenation catalyst. Optionally, stoichiometric amounts of water may be added to the mixture of methanol and formic acid. Thereby the dehydrogenation reaction proceeds in the absence of any additional hydrogen acceptor. The dehydrogenation catalyst is a transition-metal hydride catalyst or a heterogeneous transition-metal catalyst as described above.

Not falling under the protection scope of the present invention, a second aspect of the disclosure refers to a reversible chemical storage system for hydrogen construed for performing the method described above. The reversible chemical storage system for hydrogen may comprise a reactor construed for the hydrogenation step and a separate reactor construed for the dehydrogenation step according to the method described above. In another embodiment, the hydrogenation step and dehydrogenation step is performed in the same reactor. The reactor may be an autoclave or any reactor suitable for controlling the reaction temperature and the pressure conditions during dehydrogenation or hydrogenation reactions. The reversible chemical storage system for hydrogen may further comprise a hydrogen fuel cell configured to transform the hydrogen released from the dehydrogenation step into electric energy. The reversible chemical storage system for hydrogen may further comprise an electrolysis cell to provide hydrogen used for the hydrogenation step of the method described above.

A **"transition-metal hydride catalyst"** used herein refers to a transition-metal hydride complex in which at least one hydride (H) and one or two polydentate ligands (chelating ligand) coordinate to a transition-metal forming an (octahedral) complex. The catalytic activity of said transition-metal hydride catalyst may be induced by releasing one or more ligands in order to create a free coordination site at the transition-metal for coordination of the substrate, i.e. for coordination of methyl formate or water.

A **"chelating ligand"** used herein refers to a polydentate ligand, e.g. a bidentate, a tridentate or a tetradentate ligand, capable of forming a chelate complex with a (transition-) metal ion. The prefix indicates the type of chelating ligand referring to the atoms that coordinate the metal ion centre. For example, in case of a "*PNP* chelating ligand", the prefix *"PNP"* indicates the coordination sites of the ligand to the metal ion, here via a phosphor atom, a nitrogen atom and phosphor atom. Non-limiting examples may include a *PP* chelating ligand (phosphor - phosphor), *NN* chelating ligand (nitrogen - nitrogen), *PNN* chelating ligand (phosphor - nitrogen - nitrogen), *PCP* chelating ligand (phosphor - carbon - phosphor), *NCN* chelating ligand (nitrogen - carbon - nitrogen), *PCN* chelating ligand (phosphor - carbon - nitrogen), *PPP* chelating ligand (phosphor - phosphor- phosphor), *NNN* chelating ligand (nitrogen - nitrogen - nitrogen) and *PPPP* chelating ligand (phosphor - phosphor- phosphor - phosphor). Non-limiting examples for a bidentate chelating ligand may include 1,2-bis(diphenylphosphino)ethane (dppe; CAS: 1663-45-2) or 1,2-bis(dicyclohexylphosphino)ethane (CAS: 23743-26-2). Further non limiting examples for tridentate ligands may include bis(2-diphenylphosphinoethyl)phenylphosphine (Triphos²; CAS: 23582-02-7) or 1,1,1-tris(diphenylphosphinomethyl)ethane (Triphos¹; CAS: 22031-12-5). An example for a tetradentate chelating ligand may be tris[2-(diphenylphosphino)ethyl]phosphine (PP₃; CAS: 23582-03-8).

A **"hydrogen acceptor"** used herein refers to a compound that formally absorbs hydrogen (H₂) in a reaction. For instance, propylene may be reduced to propane in presence of a transition-metal hydride catalyst and hydrogen, i.e. formally absorbing hydrogen. The dehydrogenation step according to the invention proceeds, however, in the absence of a hydrogen acceptor as to release hydrogen. The term "acceptorless dehydrogenation" stems from the absence of a hydrogen acceptor.

An **"alkyl group"** used herein refers to a linear or branched aliphatic monovalent hydrocarbon group according to chemical formula CₙH₂ₙ+₁, wherein n is 1 to 18, corresponding to a C₁ -C₁₈ alkyl group. Non-limiting examples of a C₁-C₆ alkyl group may comprise n-methyl, n-ethyl, n-propyl, *iso*-propyl, *iso*-butyl, *sec*-butyl, *tert*-butyl, pentyl, *iso*-amyl and hexyl. Examples for an alkyl group with n ≥ 6 comprise n-hexyl, n-heptyl, n-octyl, n-nonyl, n-decyl, n-undecyl, n-dodecyl, n-trisdecyl, n-tetradecyl, n-pentadecyl, n-hexadecyl, n-heptadecyl, n- octadecyl, 1-methylpentyl, 1-methylhexyl, 1-methylheptyl, 1-methyloctyl, 1-methylnonyl, 1-methyldecanyl, 1-ethylbutyl, 1-ethylpentyl, 1-ethylhexyl, 1-ethylheptyl, 1-ethyloctyl, 1-ethylnonyl, 1-ethyldecanyl, 2-methylpentyl, 2-methylhexyl, 2-methylheptyl, 2-methyloctyl, 2-methylnonyl, 2-methyldecanyl, 2-ethylpropyl, 2-ethylbutyl, 2-ethylpentyl, 2-ethylhexyl, 2-ethylheptyl, 2-ethyloctyl, 2-ethylnonyl, 2-ethyldecanyl, 1,1-dimethylbutyl, 1,1-dimethylpentyl, 1,1-dimethylhexyl, 1,1-dimethylheptyl, 1,1-dimethyloctyl, 1,1-dimethylnonyl, 1,1-dimethyldecanyl, 1,2-dimethylbutyl, 1,2-dimethylpentyl, 1,2-dimethylhexyl, 1,2-dimethylheptyl, 1,2-dimethyloctyl, 1,2-dimethylnonyl, 1,2-dimethyldecanyl, 2-ethyl-1-methylbutyl, 2-ethyl-1-methylpentyl, 2-ethyl-1-methylhexyl, 2-ethyl-1-methylheptyl, 2-ethyl-1-methyloctyl, 2-ethyl-1-methylnonyl, 2-ethyl-1-methyldecanyl, 1-ethyl-2-methylpropyl, 1-ethyl-2-methylbutyl, 1-ethyl-2-methylpentyl, 1-ethyl-2-methylhexyl, 1-ethyl-2-methylheptyl, 1-ethyl-2-methyloctyl, 1-ethyl-2-methylnonyl and 1-ethyl-2-methyldecanyl.

A **"C₂-C₆ alkylene group"** used herein refers to di-radicals of the general formula CₙH₂ₙ derived from aliphatic hydrocarbons as defined above, wherein n is 2 to 6. For example, a substituent specified as an C₂-C₆ alkylene group may be a di-radical, such as -CH₂CH₂-, - CH₂CH(CH₃)CH₂- or -CH(CH₃)CH₂-.

The term **"halide"** as used herein refers to an anion derived from an element belonging to Group 17 of the periodic table, for example, fluoride, chloride, bromide, or iodide. For example, the halide may be chloride or bromide.

A "**C₃-C₇ cycloalkyl group**" used herein refers to a monovalent monocyclic saturated hydrocarbon group including 3 to 7 carbon atoms as ring-forming atoms, and non-limiting examples of the C₃-C₆ cycloalkyl group may include a cyclopropyl group, a cyclobutyl group, a cyclopentyl group, a cyclohexyl group, and a cycloheptyl group.

A "**C₅-C₇ cycloalkylene group"** used herein refers to a divalent di-radical derived from the cycloalkyl group as defined above. For example, a substituent specified as a C₅-C₇ cycloalkylene group may be a di-radical, such as cyclopentane-1,2-diyl or cyclohexane-1,2-diyl.

A **"C₆-C₁₄ aryl group"** used herein refers to a monovalent group including a carbocyclic aromatic system having 6 to 14 carbon atoms as ring-forming atoms. Non-limiting examples of the C₆-C₁₄ aryl group may include a phenyl group, a naphthyl group, an anthracenyl group and a phenanthrenyl group.

A **"C₂-C₉ heteroaryl group"** used herein refers to a monovalent group having a carbocyclic aromatic system including at least one hetero atom selected from N, O, P, and S as a ring-forming atom and 2 to 9 carbon atoms as the remaining ring-forming atoms. Non-limiting examples of the C₂-C₉ heteroaryl group may include a pyridinyl group, a pyrimidinyl group, a pyrazinyl group, a triazolyl group, an imidazolyl group, a pyridazinyl group, a triazinyl group, a quinolinyl group, an isoquinolinyl group, furanyl group, pyrrolyl and thiophenyl.

A **"C₁-C₁₈ alcohol"** used herein refers to an alcohol according to HOR with R corresponding to a C₁-C₁₈ alkyl group as defined above or C₆-C₁₄ aryl group as defined above. For example, C₁ alcohol corresponds to methanol. Further non-limiting examples of the C₁-C₁₈ alcohol may include ethanol, propanol, butanol, pentanol, hexanol, iso-propyl alcohol and iso-butyl alcohol or phenol.

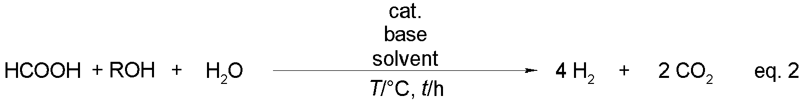

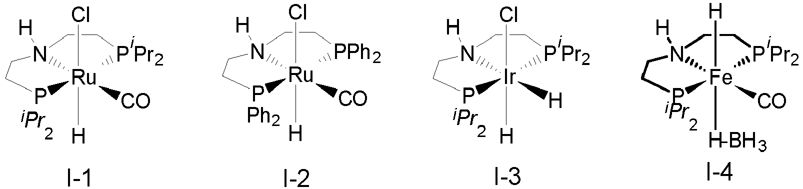

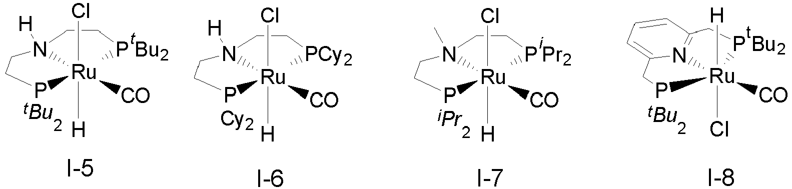

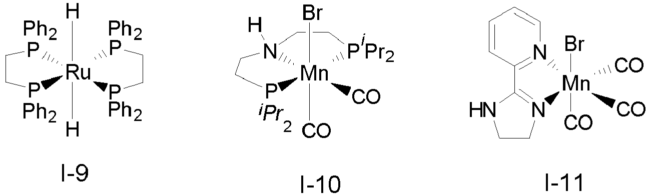

Fe(BF)₄•6H₂O/PP3 I-12

[RuCl₂(benzene)]₂/Triphos¹ I-13

[RuCl₂(benzene)]₂/Triphos² I-14

[RuCl₂(benzene)]₂/dppe I-15

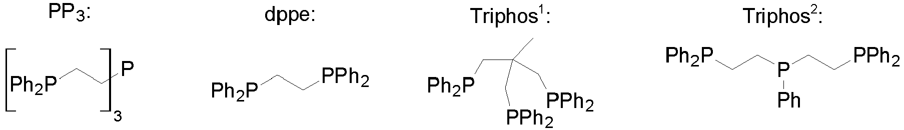

### Example 1

Acceptorless dehydrogenation of a mixture of formic acid and a C₁-C₁₈ alcohol (with R = C₁-C₁₈ alkyl) to hydrogen and carbon dioxide according to equation 2 applying selected transition-metal hydride catalysts.

The transition-metal hydride catalysts and precursors according to I-1, I-2, I-3, I-5, I-6, I-12, I-10, I-13, I-14 and I-15 were bought from fine chemical suppliers (Strem Chemicals, Sigma-Aldrich, and TCI) and used as received. Catalyst I-9 was synthesized according to a procedure published elsewhere *(*Organometallics 1997, 16, 5569-5571). Catalyst I-11 was synthesized according to a procedure published elsewhere (Green Chem. 2020, 22, 913-920). Catalyst I-7 was synthesized according to a procedure published elsewhere (J. Am. Chem. Soc. 2016, 138, 14890-14904.). Catalyst I-8 was synthesized according to a procedure published elsewhere (Angew. Chem. Int. Ed. 2010, 49, 1468-1471). Catalyst I-4 was synthesized according to a procedure published elsewhere (J. Am. Chem. Soc. 2014, 136, 7869-7872).

Formic acid and methanol were bought from fine chemical suppliers (Alfa Aesar) with 99% purity and were distilled. All substances were stored under argon atmosphere.

All experiments were carried out under argon atmosphere with the exclusion of air. If elevated temperatures were desired, a heating source was applied. The volume of evolved gases was measured by manual gas burettes (1 L or 2 L). The generation of hydrogen and carbon dioxide was verified by GC of the gas-phase. Representative reactions were reproduced to ensure the validity of results.

Under argon atmosphere 4 *µ*mol catalyst and 0.561g (10 mmol) KOH was added to a 100 mL autoclave. Then 10.0 mL triglyme, 3.2 mL formic acid (85 mmol), 3.4 mL methanol (85 mmol) and 1.5 mL H₂O (85 mmol) were injected by a syringe. After that, the autoclave was flushed with N₂ (5 bar) and the pressure was released three times. The reaction was performed at 90 °C for 20 h. Then, the autoclave was cooled to room temperature and the pressure was carefully released to a manual burette. The gas was analyzed by GC.

**Table 1: Acceptorless dehydrogenation of a mixture of formic acid and methanol to hydrogen and carbon dioxide according applying catalysts I-1 to I-3, I-8, I-15, and blank reaction.**

| **cat.** | **gas volume/mL** | **H₂/%** | **CO₂/%** | **CO/ppm** |
|---|---|---|---|---|
| I-1 | 3240 | 57.76 | 42.20 | 369 |
| I-2 | 1388 | 56.52 | 43.40 | 845 |
| I-3 | 1765 | 58.23 | 41.72 | 451 |
| I-8 | 570 | 53.27 | 46.60 | 1285 |
| I-15 | 3651 | 60.54 | 39.46 | <10 |
| without cat. | 55 | 74.31 | 21.32 | 43700 |

### Example 2

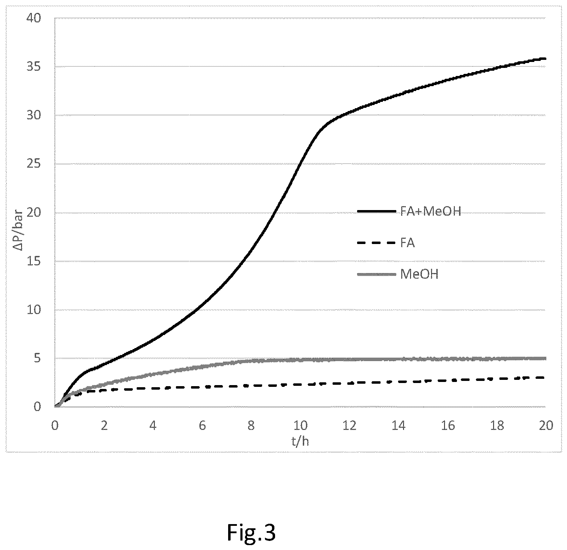

The results from figure 3 and table 2 demonstrate the improved initial reaction rate of hydrogen generation from a formic acid/formate methanol mixture compared to formic acid or formate. The reaction rate of hydrogen generation from the mixture is much faster than from formic acid or methanol alone.

Under argon atmosphere 4 *µ*mol catalyst I-1 and 0.561g (10 mmol) KOH were added to a 100 mL autoclave. Then 10.0 mL triglyme, 1.5 mL H₂O (85 mmol), 3.2 mL formic acid (85 mmol) or/and 3.4 mL methanol (85 mmol) were injected by a syringe. After that, the autoclave was flushed with N₂ (5 bar) and the pressure was released three times. The reaction was performed at 90°C for 20 hours. The autoclave was cooled to room temperature and the pressure was carefully released to a manual burette. The gas was analyzed by GC.

**Table 2: Dehydrogenation of formic acid, methanol or a mixture of formic acid and methanol applying catalyst I-1.**

| **formic acid [mmol]** | **methanol [mmol]** | **H₂O [mmol]** | **gas [volume/mL]** | **H₂ [%]** | **CO₂ [%]** | **CO [ppm]** |
|---|---|---|---|---|---|---|
| 85 | 85 | 85 | 3240 | 57.76 | 42.20 | 369 |
| 85 | without | 85 | 252 | 54.26 | 45.08 | 6638 |
| without | 85 | 85 | 498 | 79.72 | 20.28 | - |

### Example 3

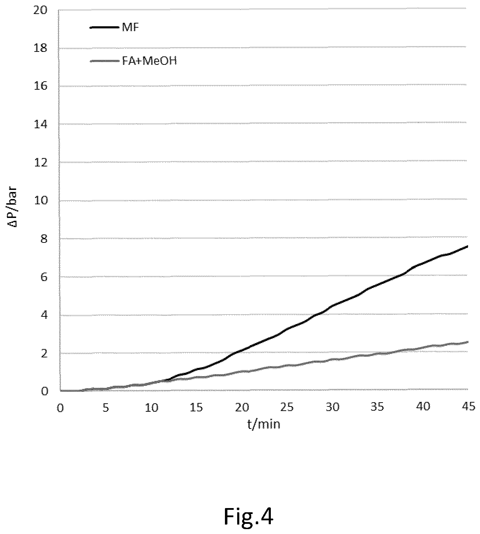

The results from figure 4 and table 3 demonstrate the generation of methyl formate from a mixture of formic acid and methanol already after 45 minutes.

Under argon atmosphere 4 *µ*mol catalyst I-1 and 0.561 g (10 mmol) KOH was added to a 100 mL autoclave. Then 10.0 mL triglyme, 3.2 mL formic acid (85 mmol), 3.4 mL methanol (85 mmol) and 1.5 mL H₂O (85 mmol) were injected by a syringe. After that, the autoclave was flushed with N₂ (5 bar) and the pressure was released three times. The reaction was performed at 90°C for 30 minutes. The autoclave was cooled to room temperature and the pressure was carefully released to a manual burette. The gas was analyzed by GC. Further the liquid phase constitution was analyzed after addition of 100 *µ*L *n*-hexadecane and 150 *µ*L propanoic acid as internal standards.

**Table 3: Activity of catalyst I-1 in the dehydrogenation of formic acid/methanol mixture compared to methyl formate and composition of the liquid phase after 45 min.**

| **substrates** | **gas volume [mL]** | **H₂ [%]** | **CO₂[%]** | **CO [ppm]** | **liquid phase** | | |
|---|---|---|---|---|---|---|---|
| | | | | | **methyl formate [mmol]** | **formic acid and formate [mmol]** | **methanol [mmol]** |
| formic acid + methanol | 131 | 57.47 | 42.53 | <10 | 19.55 | 68.01 | 61.14 |
| methyl formate | 465 | 61.21 | 38.78 | <10 | 55.15 | 22.69 | 18.51 |

### Example 4

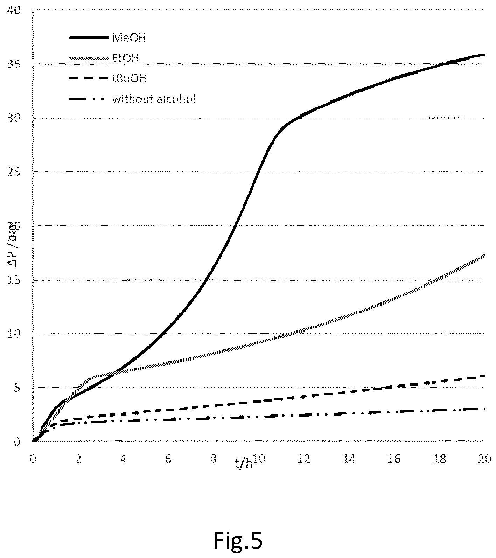

The results from figure 5 and table 4 demonstrate the dehydrogenation of formic acid/formate and alcohol mixtures in the presence of various alcohols. The reaction rate of hydrogen generation from the mixture is much faster than from the acid alone.

Under argon atmosphere 4 *µ*mol catalyst I-1 and 0.561 g (10 mmol) KOH was added to a 100 mL autoclave. Then 10.0 mL triglyme, 1.5 mL H₂O (85 mmol), 3.2 mL formic acid (85 mmol) and 85 mmol alcohol were injected by a syringe. After that, the autoclave was flushed with N₂ (5 bar) and the pressure was released three times. The reaction was performed at 90°C for 20 h. Then, the autoclave was cooled to room temperature and the pressure was carefully released to a manual burette. The gas was analyzed by GC.

**Table 4: Dehydrogenation of the of formic acid/alcohol mixtures applying different alcohols in the presence of catalyst I-1.**

| **alcohol** | **gas [mL]** | **H₂ [%]** | **CO₂ [%]** | **CO [ppm]** |
|---|---|---|---|---|
| MeOH | 3240 | 57.76 | 42.20 | 369 |
| EtOH | 1666 | 52.79 | 47.16 | 557 |
| tBuOH | 511 | 52.87 | 46.91 | 2208 |
| without | 252 | 54.26 | 45.08 | 6638 |

### Example 5

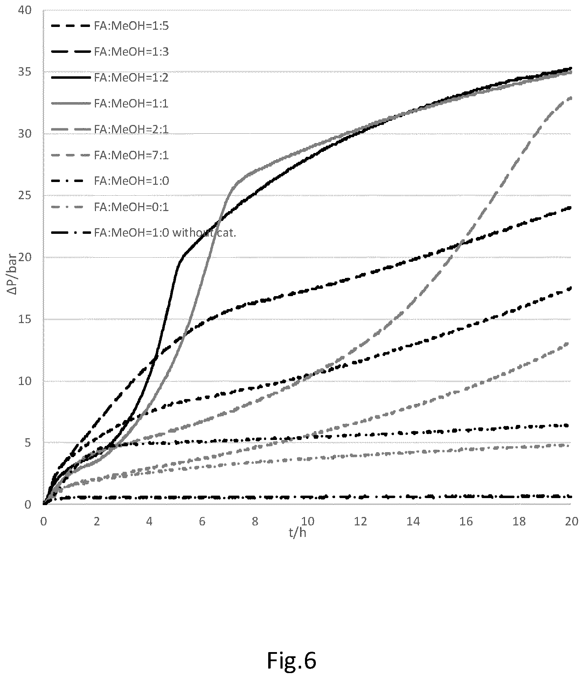

The results from figure 6 and table 5 demonstrate the dehydrogenation of formic acid/formate and methanol mixtures applying different ratios of formic acid/formate to methanol.

Under argon atmosphere 4 *µ*mol catalyst I-1 and 0.561 g (10 mmol) KOH was added to a 100 mL autoclave. Then 10.0 mL triglyme, 1.5 mL H₂O (85 mmol), 3.2 mL formic acid (85 mmol) and different amounts of methanol were injected by a syringe. After that, the autoclave was flushed with N₂ (5 bar) and the pressure was released three times. The reaction was performed at 90°C for 20 h. Then, the autoclave was cooled to room temperature and the pressure was carefully released to a manual burette. The gas was analyzed by GC.

**Table 5: Dehydrogenation of formic acid/formate and methanol mixtures applying different ratios of formic acid/formate to methanol.**

| **FA:MeOH** | **MeOH [mmol]** | **gas [mL]** | **H₂ [%]** | **CO₂ [%]** | **CO [ppm]** |
|---|---|---|---|---|---|
| 1:5 | 425 | 1425 | 58.85 | 41.15 | 67 |
| 1:3 | 225 | 2110 | 58.89 | 41.11 | <10 |
| 1:2 | 170 | 3300 | 59.51 | 40.47 | 199 |
| 1:1 | 85 | 3240 | 57.76 | 42.20 | 369 |
| 2:1 | 42.5 | 3055 | 58.45 | 41.52 | 251 |
| 7:1 | 12 | 1205 | 56.87 | 43.06 | 641 |
| 1:0 | 0 | 495 | 54.16 | 45.70 | 1422 |
| 0:1 | 85 | 375 | 86.67 | 0.13 | <10 |

### Example 6

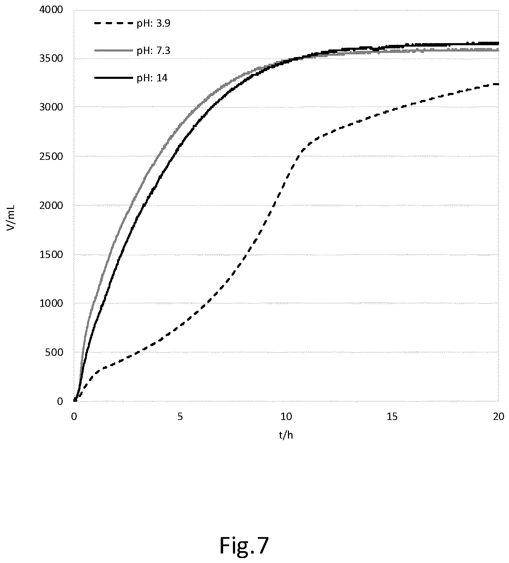

The results from figure 7 and table 6 demonstrate the dehydrogenation of formic acid/formate and methanol mixtures worlink over a broad pH range by applying different starting pH.

Under argon atmosphere 4 *µ*mol catalyst I-1 and given amount of KOH (mmol) were added to a 100 mL autoclave. Then 10.0 mL triglyme, 1.5 mL H₂O (85 mmol), 3.2 mL formic acid (85 mmol) and 3.5 mL methanol (85 mmol) were injected by a syringe. After that, the autoclave was flushed with N₂ (5 bar) and the pressure was released three times. The reaction was performed at 90°C for 20 h. Then, the autoclave was cooled to room temperature and the pressure was carefully released to a manual burette. The gas was analyzed by GC.

**Table 6: Dehydrogenation of formic acid/formate and methanol mixtures applying different starting pH.**

| **KOH [mmol]** | **pH** | **gas [mL]** | **H₂ [%]** | **CO₂ [%]** | **CO [ppm]** |
|---|---|---|---|---|---|
| 10 | 3.9 | 3240 | 57.76 | 42.20 | 369 |
| 91 | 7.3 | 3580 | 52.36 | 47.64 | <10 |
| 96 | 14 | 3660 | 51.59 | 48.41 | <10 |

### Example 7

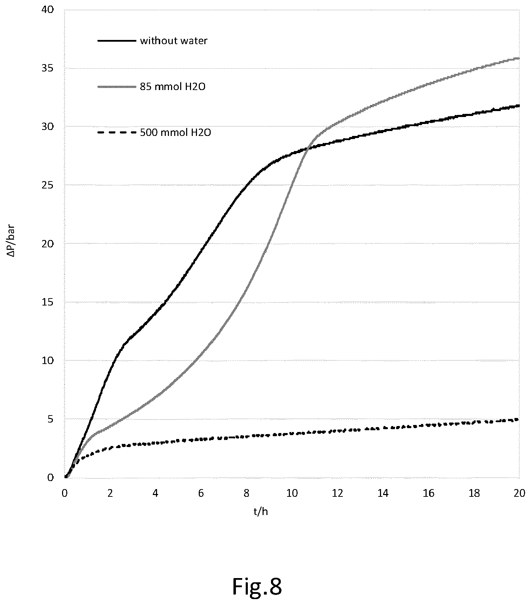

The results from figure 8 and table 7 demonstrate the dehydrogenation of formic acid/formate and methanol mixtures applying different amounts of water.

Under argon atmosphere 4 *µ*mol catalyst I-1 and 0.561 g (10 mmol) mmol KOH was added to a 100 mL autoclave. Then 10.0 mL triglyme, the given amount of H₂O (mmol), 3.2 mL formic acid (85 mmol) and 3.5 mL methanol (85 mmol) were injected by a syringe. After that, the autoclave was flushed with N₂ (5 bar) and the pressure was released three times. The reaction was performed at 90°C for 20 h. Then, the autoclave was cooled to room temperature and the pressure was carefully released to a manual burette. The gas was analyzed by GC.

**Table 7: Dehydrogenation of formic acid/formate and methanol mixtures applying different amount of water.**

| **H₂O [mmol]** | **gas [mL]** | **H₂ [%]** | **CO₂ [%]** | **CO [ppm]** |
|---|---|---|---|---|
| without | 2976 | 59.03 | 40.96 | 160 |
| 85 | 3240 | 57.76 | 42.20 | 369 |
| 500 | 318 | 54.04 | 45.80 | 1677 |

## Claims

1. A method for the reversible chemical storage of hydrogen, the method comprising:
1) a hydrogenation step, which comprises contacting hydrogen with carbon dioxide in the presence of a hydrogenation catalyst to effect storage of hydrogen by forming at least formic acid; and
2) a dehydrogenation step, which comprises converting a mixture comprising or consisting of a C₁-C₁₈ alcohol and formic acid into hydrogen and carbon dioxide in the presence of a base and
a) a transition-metal hydride catalyst or precursor thereof, the transition-metal hydride catalyst comprising:
a metal-ion selected from the group consisting of Ru, Ir, Mn, Co, Mo, Fe and Rh,
at least one hydride ligand coordinated to the metal-ion, and
i) a tridentate ligand coordinated to the metal-ion and selected from the group consisting of a *PNP*-chelating ligand, a *PNN*-chelating ligand, a *PCP*-chelating ligand, an *NCN-*chelating ligand, a *PCN*-chelating ligand, a *PPP*-chelating ligand and an *NNN*-chelating ligand, or
ii) one or two a bidentate ligand coordinated to the metal-ion and selected from a *PP-*chelating ligand or an *NN-*chelating ligand, or
iii) a tetradentate *PPPP-*chelating ligand coordinated to the metal-ion; or
b) a heterogeneous transition-metal catalyst.

2. The method according to claim 1, wherein the C₁-C₁₈ alcohol is a C₁-C₆ alcohol.

3. The method according to claims 1 or 2, wherein the metal-ion is selected from the group consisting of Ru, Ir, Fe and Mn.

4. The method according to one of the preceding, wherein the tridentate ligand of the transition-metal hydride catalyst is a *PNP*-chelating ligand according to formula (II): 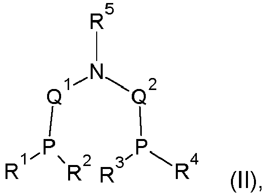 with
Q¹, Q² independently selected from the group consisting of C₂-C₆ alkylene group and C₅-C₇ cycloalkylene group;
R¹, R², R³, R⁴ independently selected from the group consisting of C₁-C₆ alkyl group, C₃-C₇ cycloalkyl group, C₆-C₁₄ aryl group, C₂-C₉ heteroaryl group and benzyl; and
R⁵ independently selected from the group consisting of from H, C₁ - C₈ alkyl group and C₃-C₇ cycloalkyl group.

5. The method according to claim 1, wherein the bidentate ligand is 1,2-bis(diphenylphosphino)ethane.

6. The method according to claim 1, wherein the transition-metal hydride catalyst is selected from at least one of the group consisting of the following compounds:

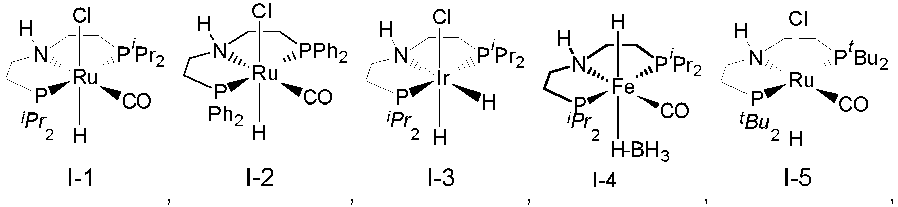

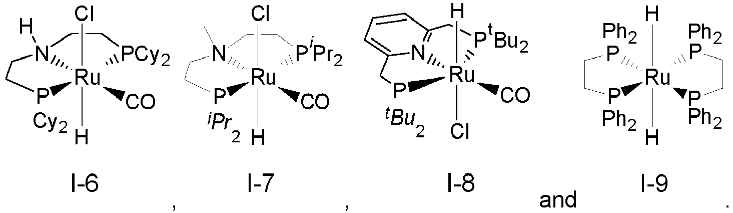

7. The method according to claim 1, wherein the precursor of the transition-metal hydride catalyst is selected from at least one of the group consisting of the following compounds: 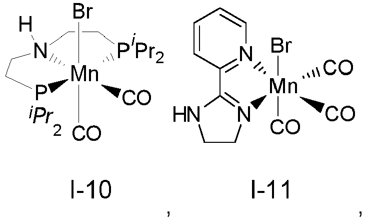
Fe(BF)₄•6H₂O/PP3 I-12 ,
[RuCl₂(benzene)]₂/Triphos¹ I-13 ,
[RuCl₂(benzene)]₂/Triphos² I-14
and
[RuCl₂(benzene)]₂/dppe I-15 ,
wherein

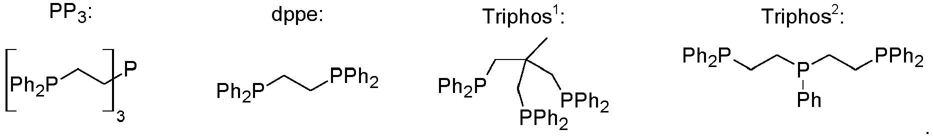

8. The method according to one of the preceding claims, wherein the transition-metal hydride catalyst or precursor thereof is present at 0.001 mol% to 0.01 mol% referred to formic acid.

9. The method according to claim 1, wherein the heterogeneous transition-metal catalyst comprises a metal selected from at least one of the group consisting of ruthenium, palladium, platinum and cobalt.

10. The method according to one of the preceding claims, wherein the base is selected from at least one of the group consisting of KOH, NaOH, CsOH, LiOH and K₃PO₄.

11. The method according to one of the preceding claims, wherein the base is present at 5 mol% to 35 mol% referred to formic acid.

12. The method according to one of the preceding claims, wherein the molar ratio of the mixture according to formic acid : C₁-C₁₈ alcohol: water is 0.9 to 1.1 : 0.5 to 2 : 0 to 2.

13. The method according to one of the preceding claims, wherein the dehydrogenation conditions comprise a reaction temperature of 20°C to 150°C.

14. The method according to claim 1, wherein the hydrogenation catalyst corresponds to the transition-metal hydride catalyst or to the heterogeneous transition-metal catalyst according to claim 1.

## Patentansprüche

1. Verfahren zur reversiblen chemischen Speicherung von Wasserstoff, wobei das Verfahren umfasst:
1) einen Hydrierungsschritt, der das Inkontaktbringen von Wasserstoff mit Kohlendioxid in Gegenwart eines Hydrierungskatalysators umfasst, um die Speicherung von Wasserstoff durch Bildung von mindestens Ameisensäure zu bewirken; und
2) einen Dehydrierungsschritt, der die Umwandlung eines Gemischs, das einen C₁-C₁₈⁻ Alkohol und Ameisensäure umfasst oder daraus besteht, in Wasserstoff und Kohlendioxid in Gegenwart einer Base umfasst, und
a) einen Übergangsmetallhydrid-Katalysator oder einen Vorläufer davon, wobei der Übergangsmetallhydrid-Katalysator umfasst:
ein Metallion, ausgewählt aus der Gruppe bestehend aus Ru, Ir, Mn, Co, Mo, Fe und Rh,
mindestens einen an das Metallion koordinierten Hydrid-Liganden und
i) einen dreizähnigen Liganden, der an das Metallion koordiniert ist und aus der Gruppe ausgewählt ist, die aus einem *PNP-chelatbildenden* Liganden, einem *PNN-chelatbildenden* Liganden, einem *PCP-chelatbildenden* Liganden, einem *NCN-chelatbildenden* Liganden, einem *PCN-chelatbildenden* Liganden, einem *PPP-chelatbildenden* Liganden und einem *NNN-chelatbildenden* Liganden besteht, oder
ii) einen oder zwei zweizähnigen Liganden, die an das Metallion koordiniert sind und aus einem *PP-chelatierenden* Liganden oder einem *NN-chelatierenden* Liganden ausgewählt sind, oder
iii) einen vierzähnigen *PPPP-chelatierenden* Liganden, der an das Metallion koordiniert ist; oder
b) einen heterogenen Übergangsmetallkatalysator.

2. Verfahren nach Anspruch 1, wobei der C₁-C₁₈-Alkohol ein C₁-C₆-Alkohol ist.

3. Verfahren nach Anspruch 1 oder 2, wobei das Metallion ausgewählt ist aus der Gruppe bestehend aus Ru, Ir, Fe und Mn.

4. Verfahren nach einem der vorhergehenden Ansprüche, wobei der dreizähnige Ligand des Übergangsmetallhydridkatalysators ein PNP-Chelatligand der Formel (II) ist: 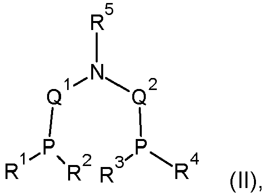 wobei
Q¹, Q² unabhängig ausgewählt sind aus der Gruppe bestehend aus C₂-C₆-Alkylengruppe und C -C₇-Cycloalkylengruppe;
R¹, R², R³, R⁴ unabhängig ausgewählt sind aus der Gruppe bestehend aus C₁-C₆-Alkylgruppe, C₃-C₇-Cycloalkylgruppe, C -C₁₄-Arylgruppe, C₂-C₉-Heteroarylgruppe und Benzyl; und
R⁵ unabhängig ausgewählt ist aus der Gruppe bestehend aus H, C₁- C₈-Alkylgruppe und C₃- C₇.Cycloalkylgruppe.

5. Verfahren nach Anspruch 1, wobei der zweizähnige Ligand 1,2-Bis(diphenylphosphino)ethan ist.

6. Verfahren nach Anspruch 1, wobei der Übergangsmetallhydrid-Katalysator aus mindestens einer der folgenden Verbindungen ausgewählt ist:

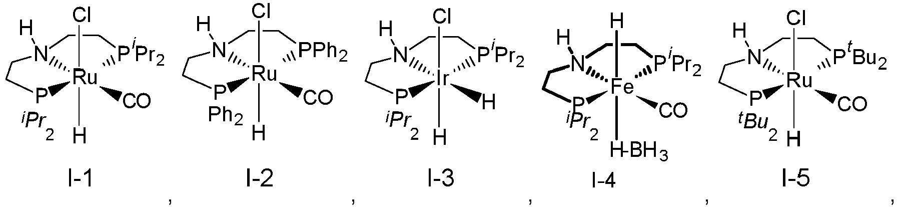

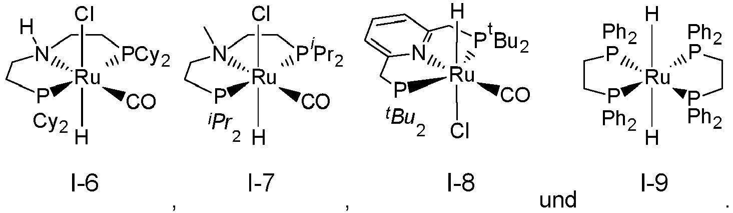

7. Verfahren nach Anspruch 1, wobei der Vorläufer des ÜbergangsmetallhydridKatalysators aus mindestens einer der Gruppe bestehend aus den folgenden Verbindungen ausgewählt ist: 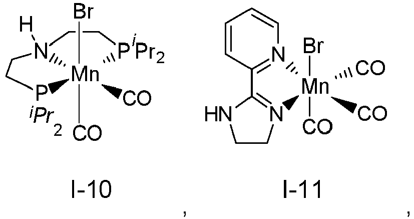
Fe(BF)₄•6H₂O/PP3 I-12
[RuCl₂(benzene)]₂/Triphos¹ I-13
[RuCl₂(benzene)]₂/Triphos² I-14
and
[RuCl₂(benzene)]₂/dppe I-15
wobei

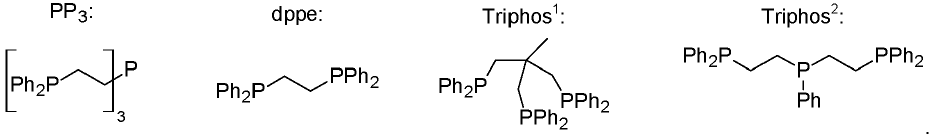

8. Verfahren nach einem der vorhergehenden Ansprüche, wobei der Übergangsmetallhydrid-Katalysator oder dessen Vorläufer in einer Menge von 0,001 Mol-% bis 0,01 Mol-%, bezogen auf Ameisensäure, vorliegt.

9. Verfahren nach Anspruch 1, wobei der heterogene Übergangsmetallkatalysator ein Metall umfasst, das aus mindestens einer der Gruppe bestehend aus Ruthenium, Palladium, Platin und Kobalt ausgewählt ist.

10. Verfahren nach einem der vorhergehenden Ansprüche, wobei die Base aus mindestens einer der Gruppe bestehend aus KOH, NaOH, CsOH, LiOH und K₃PO₄ ausgewählt ist.

11. Verfahren nach einem der vorhergehenden Ansprüche, wobei die Base in einer Menge von 5 bis 35 Mol-%, bezogen auf Ameisensäure, vorhanden ist.

12. Verfahren nach einem der vorhergehenden Ansprüche, wobei das molare Verhältnis der Mischung gemäß Ameisensäure : C₁-C₁₈-Alkohol : Wasser 0,9 bis 1,1 beträgt: 0,5 bis 2 : 0 bis 2 beträgt.

13. Verfahren nach einem der vorhergehenden Ansprüche, wobei die Dehydrierungsbedingungen eine Reaktionstemperatur von 20°C bis 150°C umfassen.

14. Verfahren nach Anspruch 1, wobei der Hydrierungskatalysator dem Übergangsmetallhydridkatalysator oder dem heterogenen Übergangsmetallkatalysator nach Anspruch 1 entspricht.

## Revendications

1. Procédé de stockage chimique réversible de l'hydrogène, comprenant :
1) une étape d'hydrogénation, qui comprend mettre en contact de l'hydrogène avec du dioxyde de carbone en présence d'un catalyseur d'hydrogénation pour stocker l'hydrogène en formant au moins de l'acide formique ; et
2) une étape de déshydrogénation, qui comprend convertir un mélange comprenant ou consistant en un alcool C₁-C₁₈ et de l'acide formique en hydrogène et en dioxyde de carbone en présence d'une base et
a) un catalyseur d'hydrure de métal de transition ou son précurseur, le catalyseur d'hydrure de métal de transition comprenant :
un ion métallique choisi dans le groupe constitué par Ru, Ir, Mn, Co, Mo, Fe et Rh,
au moins un ligand hydrure coordonné à l'ion métallique, et
i) un ligand tridenté coordonné à l'ion métallique et choisi dans le groupe constitué d'un ligand *chélateur PNP,* d'un ligand *chélateur PNN,* d'un ligand *chélateur PCP,* d'un ligand chélateur *NCN*, d'un ligand chélateur *PCN*, d'un ligand chélateur *PPP* et d'un ligand *chélateur NNN,* ou
ii) un ou deux ligands bidentés coordonnés à l'ion métallique et choisis parmi un ligand *chélateur PP* ou un ligand *chélateur NN,* ou
iii) un ligand *chélateur PPPP* tétradenté coordonné à l'ion métallique ; ou
b) un catalyseur hétérogène à base de métal de transition.

2. Procédé selon la revendication 1, dans lequel l'alcool C₁-C₁₈ est un alcool C₁-C _{6.}

3. Procédé selon les revendications 1 ou 2, dans lequel l'ion métallique est choisi dans le groupe constitué par Ru, Ir, Fe et Mn.

4. Procédé selon l'un des procédés précédents, dans lequel le ligand tridenté du catalyseur d'hydrure de métal de transition est un ligand *chélateur de PNP* selon la formule (II) : 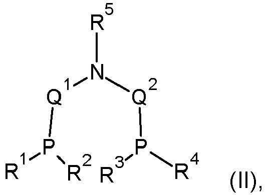 où
Q¹, Q² sont choisis indépendamment dans le groupe constitué par le groupe alkylène C₂-C₆ et le groupe cycloalkylène C₅-C₇ ;
R¹, R², R³, R⁴ sont choisis indépendamment dans le groupe constitué par le groupe alkyle C₁-C₆, le groupe cycloalkyle C₃-C₇, le groupe aryle C₆-C₁₄, le groupe hétéroaryle C₂-C₉ et le benzyle ; et
R⁵ sont indépendamment choisi dans le groupe constitué de H, C₁-C₈ groupe alkyle et C₃-C₇ groupe cycloalkyle.

5. Procédé selon la revendication 1, dans lequel le ligand bidenté est le 1,2-bis(diphénylphosphino)éthane.

6. Procédé selon la revendication 1, dans lequel le catalyseur d'hydrure de métal de transition est choisi parmi au moins l'un du groupe constitué des composés suivants :

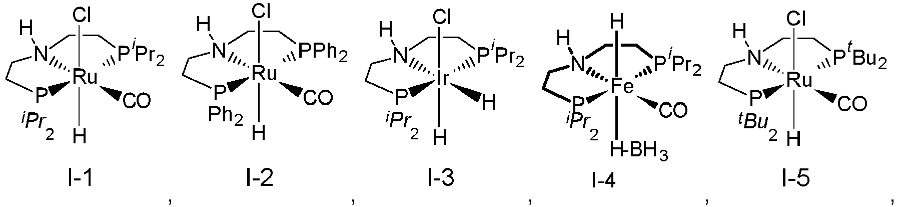

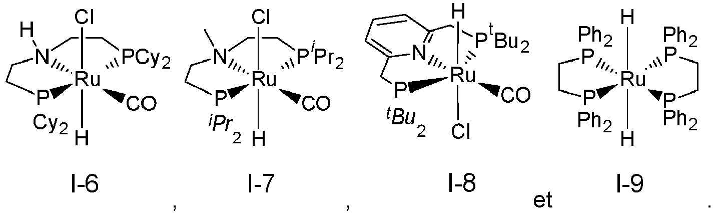

7. Procédé selon la revendication 1, dans lequel le précurseur du catalyseur d'hydrure de métal de transition est choisi dans au moins l'un du groupe constitués par les composés suivants : 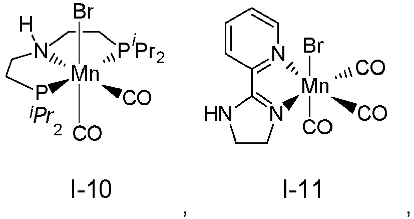
Fe(BF)₄•6H₂O/PP3 I-12 ,
[RuCl₂(benzene)]₂/Triphos¹ I-13 ,
[RuCl₂(benzene)]₂/Triphos² I-14
et
[RuCl₂(benzene)]₂/dppe I-15 ,
dans lequel

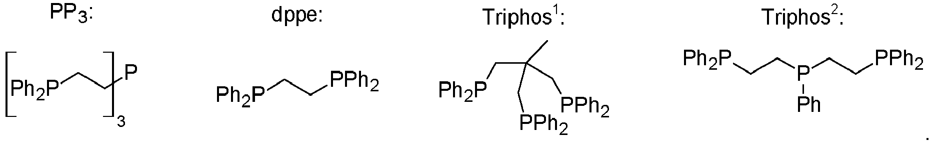

8. Procédé selon l'une des revendications précédentes, dans lequel le catalyseur d'hydrure de métal de transition ou son précurseur est présent à raison de 0,001 mol% à 0,01 mol% par rapport à l'acide formique.

9. Procédé selon la revendication 1, dans lequel le catalyseur hétérogène à base de métal de transition comprend un métal choisi dans au moins l'un du groupe constitué de ruthénium, palladium, platine et cobalt.

10. Procédé selon l'une des revendications précédentes, dans lequel la base est choisie dans au moins l'un du groupe constitué de KOH, NaOH, CsOH, LiOH et K₃PO₄.

11. Procédé selon l'une des revendications précédentes, dans lequel la base est présente à raison de 5 mol% à 35 mol% par rapport à l'acide formique.

12. Procédé selon l'une des revendications précédentes, dans lequel le rapport molaire du mélange selon l'acide formique : alcool C₁-C₁₈ : eau est de 0,9 à 1,1 : 0,5 à 2 : 0 à 2.

13. Procédé selon l'une des revendications précédentes, dans lequel les conditions de déshydrogénation comprennent une température de réaction de 20°C à 150°C.

14. Procédé selon la revendication 1, dans lequel le catalyseur d'hydrogénation correspond au catalyseur d'hydrure de métal de transition ou au catalyseur hétérogène à base de métal de transition selon la revendication 1.
